# EUROPEAN PATENT SPECIFICATION

(11) **EP 3 171 920 B1**
(45) Date of publication and mention of the grant of the patent: **29.04.2020**
(21) Application number: 15729036.2
(22) Date of filing: 03.06.2015
(51) Int. Cl.: A61M 11/00, A61M 15/00, B05B 17/06

(54) **DRY POWDER NEBULIZER**
TROCKENPULVERINHALATOR
NÉBULISEUR DE POUDRE SÈCHE

(30) Priority: 23.07.2014 US 201462028172 P
(43) Date of publication of application: 31.05.2017
(73) Proprietor: MicroDose Therapeutx, Inc., Ewing, NJ 08628 (US)
(72) Inventor: MORRISON, Mark, Steven, Basking Ridge, New Jersey 07920 (US)
(74) Representative: Elkington and Fife LLP
(86) International application number: PCT/US2015/034050
(87) International publication number: WO 2016/014154

(56) References cited:
- EP-A1- 1 005 917
- EP-A1- 1 950 421
- US-A- 3 474 967
- US-A- 4 981 425
- US-A1- 2006 213 503
- US-A1- 2008 156 320
- US-A1- 2008 202 514
- US-A1- 2010 229 860

## Description

The present invention relates generally to the field of metering, packaging and delivery of pharmaceuticals and drugs. Particular utility for the present invention is found in delivery of metered and packaged dry powder medications and drugs for inhalation therapy and will be described in connection with such utility, although other utilities are contemplated, including liquid medication applications.

Certain diseases of the respiratory tract are known to respond to treatment by the direct application of therapeutic agents. As these agents are most readily available in dry powdered form, their application is most conveniently accomplished by inhaling the powdered material through the nose or mouth. This powdered form results in the better utilization of the medication in that the drug is deposited exactly at the site desired and where its action may be required; hence, very minute doses of the drug are often equally as efficacious as larger doses administered by other means, with a consequent marked reduction in the incidence of undesired side effects and medication cost. Alternatively, the drug in powdered form may be used for treatment of diseases other than those of the respiratory system. When the drug is deposited on the very large surface areas of the lungs, it may be very rapidly absorbed into the blood stream; hence, this method of application may take the place of administration by injection, tablet, or other conventional means.

It is the opinion of the pharmaceutical industry that the bioavailability of the drug is optimum when the drug particles delivered to the respiratory tract are between 1 to 5 microns in size. When the drug particles need to be in this size range the dry powder delivery system needs to address a number of issues:
(1) Small size particles develop an electrostatic charge on themselves during manufacturing and storage. This causes the particles to agglomerate or aggregate, resulting in clusters of particles which have an effective size greater than 5 microns. The probability of these large clusters making it to the deep lungs then decreases. This in turn results in a lower percentage of the drug being available to the patient for absorption.
(2) The amount of active drug that needs to be delivered to the patient may be of the order of tens of micrograms. Since current powder filling equipment cannot effectively deliver aliquots of drugs in microgram quantities with acceptable accuracy, the standard practice is to mix the active drug with a filler or bulking agent such as lactose. This additive also makes the drug "easy to flow". In some cases this filler is sometimes called a carrier. These carrier particles are often larger than the drug particles in size. The ability of the dry powder inhaler to separate drug from the carrier is an important performance parameter in the effectiveness of the design.
(3) Active drug particles with sizes greater than 5 microns will be deposited either in the mouth or throat. This introduces another level of uncertainty since the bioavailability and absorption of the drug in these locations is different from the lungs. Dry powder inhalers need to minimize the drug deposited in these locations to reduce the uncertainty associated with the bioavailability of the drug.

Prior art dry powder inhalers (DPIs) usually have a means for introducing the drug (active drug plus carrier) into a high velocity air stream. The high velocity air-stream is used as the primary mechanism for breaking up the cluster of micronized particles or separating the drug particles from the carrier. Several inhalation devices useful for dispensing this powder form of medication are known in the prior art. For example, in U.S. Pat. Nos. 3,507,277; 3,518,992; 3,635,219; 3,795,244; and 3,807,400, inhalation devices are disclosed having means for piercing or removing the top of a capsule containing a powdered medication, which upon inhalation is drawn out of the pierced or topped capsule and into the user's mouth. Several of these patents disclose propeller means, which upon inhalation aid in dispensing the powder out of the capsule, so that it is not necessary to rely solely on the inhaled air to suction powder from the capsule.
Prior art devices such as above described have a number of disadvantages which makes them less than desirable for the delivery of dry powder to the lungs. Some of these disadvantages include:
- The performance of the prior art inhalers depends on the flow rate generated by the user. Lower flow rate does not result in the powder being totally deaggregated and hence adversely affects the dose delivered to the patient.
- Inconsistency in the bioavailability of the drugs from dose-to-dose because of lack of consistency in the deaggregation process.
- Large energy requirements for driving the electromechanical based inhalers which increases the size of the devices making them unsuitable for portable use.
- Loss of medication from opened or topped capsules.
- Deterioration of medication in open or topped capsule due to exposure to oxygen or moisture.

The foregoing discussion of the prior art derives in part from U.S. Patent 7,318,434, in which there is described a dry powder inhaler which employs synthetic jetting technology to aerosolize drug powder from a blister pack or the like. It is known that if one uses a chamber bounded on one end by an acoustic wave generating device and bounded on the other end by a rigid wall with a small orifice, that when acoustic waves are emitted at high enough frequency and amplitude from the generator, a jet of air that emanates from the orifice outward from the chamber can be produced. The jet, or so-called "synthetic jet", is comprised of a train of vortical air puffs that are formed at the orifice at the generator's frequency. However, as described in the aforesaid '434 patent, the use of a synthetic jet to deaggregate and eject a dry-powder material from a blister pack or the like provides advantages over prior art dry powder inhalers.

More particularly, the aforesaid '434 patent provides a dry powder inhaler having a first chamber for and holding a dry powder, and a second chamber connected to the first chamber via a passageway for receiving an aerosolized form of the dry powder from the first chamber and for delivering the aerosolized dry powder to a user. A vibrator is coupled to the dry powder in the first chamber. Since jetting efficiency falls off as the aspect ratio (length to cross-section or diameter) of the passageway increases, in order to create a synthetic jet the passageway connecting the first chamber to the second chamber preferably, but not necessarily has an aspect ratio equal to at least about one, and the vibrator is energized and coupled to the first chamber so that the distance the gas moves back and forth in the passageway is at least about twice the cross-section or diameter of the passageway.

In one embodiment of the aforesaid '434 patent, the first chamber is formed in the shape of a cylinder or blister with a vibratory element either forming one wall of the chamber, or the vibratory element is formed apart from the chamber and coupled to the blister.

In a second embodiment of the aforesaid '434 patent the first chamber is formed in the shape of a horn, with a vibratory element either forming one wall of the chamber, or the vibratory element is coupled to a wall of the chamber via a column of gas.

In a third embodiment the aforesaid '434 patent the first chamber is formed in the shape of a horn, and a standing wave resonator is coupled to a wall of the chamber. See also U.S. Patent Nos. 7,334,577; 7,779,837 and 8,322,338. US 2008/0202514 A1 discloses a dry powder blister using synthetic jetting.

The blister implementation described by the aforementioned patents bears some resemblance to an inverted kettle drum, whereby a piezoelectric transducer applies acoustic energy to the open end of the chamber (i.e. drum). Small holes at the closed end provide an escape path for drug loaded in the chamber. When driven at the right frequency, as governed by dimensions of both the piezo and the chamber, a unique standing wave pattern is created that, owing to the unique shape of the chamber, conveniently places pressure anti-nodes at both ends, with a pressure node in between.

The pressure anti-node nearest the closed end of the chamber works in concert with the small holes at that end to create synthetic jets that expel drug from the chamber.

Synthetic jetting is the phenomenon by which air passing rapidly through an opening develops vortices that move away from the opening. The same thing happens in the opposite direction, at different times, such that the net air mass flow is zero. These 'internal vortices' (or jets) assist with mixing of powder within the chamber. However, the vortices leaving the chamber carry with them powdered drug, which leaves the chamber and does not return. These are the particles available for patient inhalation.

In one aspect of the invention there is provided an inhalation device comprising a drug chamber for holding a dry powder pharmaceutical;
a vibrator for aerosolizing said pharmaceutical within the drug chamber; and
a wave reflector, wherein the drug chamber comprises at least one inlet hole and at least one outlet hole via which aerosolized pharmaceutical may be ejected; and
wherein the device is configured such that a standing wave pattern is created by acoustic energy that is produced by the vibrator and reflected back at least in part to the vibrator by the reflector so that air can be drawn into the inlet hole and out from the outlet hole, wherein acoustic energy produced by the vibrator is sufficient to create a flow within the drug chamber capable of carrying the pharmaceutical out of the outlet and,
wherein the at least one inlet hole is located adjacent to a node of the standing wave pattern, and/or the at least one outlet hole is located adjacent to an antinode of the standing wave pattern.

In one embodiment, the acoustic waves are produced by a piezoelectric transducer.

With the present invention it is possible to deliver a drug for inhalation in a simple and reliable way with a device which is low cost yet capable of effective delivery. It also enables a device which is compact and with low power requirements.

Examples of the present invention will now be described with reference to the accompanying drawings, in which:
Figures 1 and 2 are schematic side views of examples of prior art arrangements; and
Figures 3 and 4 are schematic side views of components of a device according to the invention.

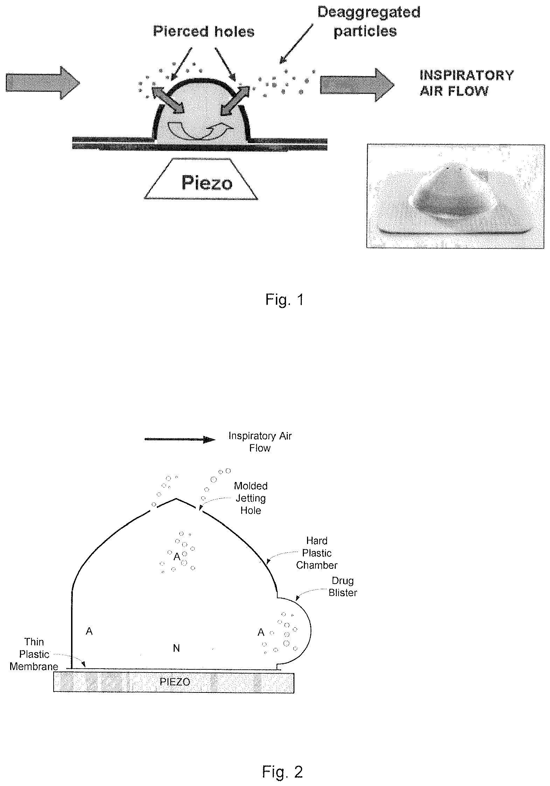

Referring to figure 1, a known design uses a special dome shaped drug blister as the chamber. This requires a special piercing tool to create the jetting holes just prior to use. In this case, the piezo is placed in contact with the lidding material of the sealed blister, vibrating the bottom of the blister and causing direct agitation of the drug powder within. In this capacity, the piezo 1) creates the acoustic waves that result in synthetic jetting, and 2) deagglomerates the drug resting on the lid material by direct vibration.

More recently, an alternative has been designed, and is a drug delivery system comprising a dose chamber coupled to a vibrating device as described in U.S. Application 12/985,158.

In an embodiment described in the '158 application, an inhaler is provided with a combined reservoir and dosing chamber configured to receive multiple doses of a pharmaceutical material. As before, the dosing chamber is coupled to a vibration device for aerosolizing the pharmaceutical, and delivering aerosolized pharmaceuticals to the patient.

Even more recently, the hard dosing chamber described in the '158 patent has been modified to include a thin membrane that serves to both seal off the dosing chamber as well as couple the chamber to the vibrating device as illustrated in figure 2 (note that A stands for pressure antinode, and N stands for pressure node).

As can be seen, a thin plastic film now covers the open end, through which the piezo applies acoustic energy. Small jetting holes are molded into the chamber, replacing those created in the original design by way of piercing. In this case, the drug blister itself has been relocated to the side of the chamber, where its contents are delivered to the chamber through a small opening in the chamber wall, as a result of the lidding material being peeled back. In this position, the opening of the blister is placed in close proximity to a pressure antinode (A) on the outer circumference of the chamber. The transport of drug from the blister to the chamber is thought to be facilitated by pressure variations at the antinode as well as direct vibration of the piezo coupled into the blister by way of the surrounding structure, which is in communication with the piezo.

It should be noted that all of the previous descriptions employ synthetic jetting to transport powdered drug to the patient for inhalation. The present invention, on the other hand, uses using acoustic streaming to disperse the powder pharmaceutical. Acoustic streaming is the phenomenon by which sound travelling through a medium imparts momentum to that medium, causing it to move. One example is so called 'Rayleigh Streaming', which can be demonstrated using a 40 kHz piezo transducer. For example, if such a transducer is driven at sufficiently high amplitude facing into a closed tube with a suitable reflector at the opposite end, it is possible to displace powders within the tube. Surprisingly, this effect is more than adequate to aerosolize deagglomerated fine powders. This effect may also deagglomerate some drug pellets, as would be understood by a person skilled in the art.

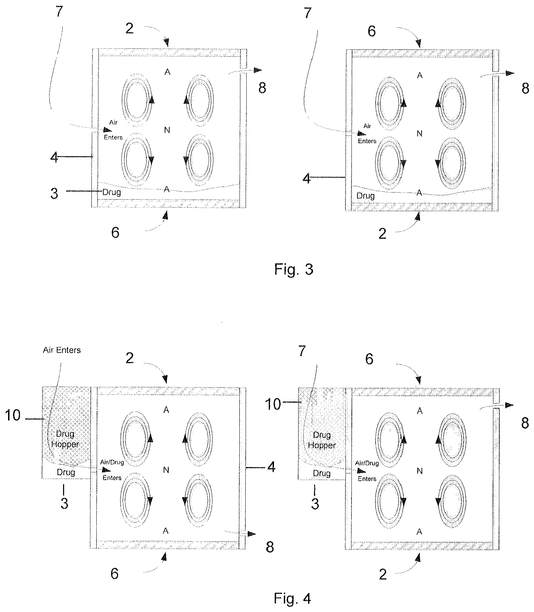

As shown in Figure 3, the inventors have surprisingly found that if a reflector 6 is introduced, such that most of the waves are returned directly to the transducer, it is possible to create a standing wave pattern within the chamber. If such a chamber is a simple tube shape, with the piezo 2 closing off one end, and a hard reflector at the other, pressure nodes and antinodes are developed at very specific locations. By placing holes 7, 8 in the chamber walls 4 at these locations, a node serves as a pump inlet while a pressure antinode serves as a pump outlet. If driven with sufficient acoustic energy, acoustic non-linearities form within the sound field, causing a pressure swing at the anti-node to become asymmetric, resulting in a pressure differential that is sufficient to create flow. Note that the piezo 2 can be placed at either end of the tube, but better deagglomeration is possible with the piezo placed at the bottom of the tube where it can assist with deagglomeration by means of direct vibration of the drug powder.

When dry powder is introduced into the system, which can be done by any convenient means (not illustrated), the powder may follow the path of air being pumped through the system. This is shown in Figures 3 and 4 where an example of a dry powder nebulizer based upon the "standing wave pump" is illustrated. In this scenario, the piezo 2 (PZT) serves not only to create the required acoustic waves, but also to actively vibrate the powdered drug 3. We note that this pumping action does not involve synthetic jetting.

An alternative configuration is illustrated in Figure 4. In this case, drug 3 is contained inside a hopper 10 that is in fluid communication with a pressure node. Drug is "metered" out of the hopper 10 into the standing wave pump where it finds its way out the pressure antinode at the bottom. In this case, vibrations of the piezo 2 couple directly into the drug hopper, agitating particles and causing them to move into the chamber.

Fine particles can become trapped within pressure nodes, coming to rest under the force of gravity at the junction of that node and the antinode directly below it. This may otherwise be considered disadvantageous but, on the contrary, it is possible to exploit this effect for the purpose of metering out discrete packets of powdered drug. This would be particularly advantageous for the nebulizer illustrated above, which does not have drug blisters to provide such metering. Particles can be trapped in small packets and then caused to move toward some convenient exit point where they may be released from the system. It is possible to achieve this by changing the frequency of piezo operation, while being careful not to operate the piezo off resonance, where output would otherwise drop considerably. Such embodiments are included in the present invention.

In another embodiment, a further means of accomplishing the same thing is to use a reflector 6 with acoustic impedance that results in partial reflection, in which case some of the acoustic energy travels through the reflector, and some is reflected. The weaker reflected waves can provide a different, possibly changing, interference pattern that causes the nodes and anti-nodes to move. The advantage of this is that there is no requirement to change frequency on the fly, something that greatly simplifies the device. Also, this can facilitate using a drug reservoir approach, as opposed to a blister approach, providing further benefit by eliminating the drug strip, motor and related sensor. Of course a motor might still be required in order to bring the drug within proximity of the (acoustic) driving source.

It should be noted that only particles of a certain size tend to become trapped within the pressure nodes. Those too heavy to be supported by the acoustic field fall back to the drug load where they can be further agitated. Those that are light enough to be supported can be used for inhalation.

It should be noted that although a drug hopper is shown in figure 4, any means of delivering powder to the chamber could be used instead, including but not limited to drug blisters.

It should be understood that the foregoing detailed description and preferred embodiments are only illustrative of inhalation devices constructed in accordance with the present disclosure. Various alternatives and modifications to the presently disclosed inhalers can be devised by those skilled in the art without departing from the scope of the present disclosure.

## Claims

1. An inhalation device comprising a drug chamber (4) for holding a dry powder pharmaceutical (3);
a vibrator (2) for aerosolizing said pharmaceutical (3) within the drug chamber (4); and
a wave reflector (6), wherein the drug chamber (4) comprises at least one inlet hole (7) and at least one outlet hole (8) via which aerosolized pharmaceutical may be ejected; and wherein the device is configured such that a standing wave pattern is created by acoustic energy that is produced by the vibrator (2) and reflected back at least in part to the vibrator (2) by the reflector (6) so that air can be drawn into the inlet hole (7) and out from the outlet hole (8), wherein acoustic energy produced by the vibrator (2) is sufficient to create a flow within the drug chamber (4) capable of carrying the pharmaceutical (3) out of the outlet hole (8), and
**characterized in that** the at least one inlet hole (7) is located adjacent to a node of the standing wave pattern, and/or the at least one outlet hole (8) is located adjacent to an antinode of the standing wave pattern.

2. The device of claim 1, wherein the vibrator (2) comprises a piezoelectric transducer.

3. The device of claim 1 or 2, wherein the drug chamber (4) is tube-shaped, and/or, wherein the vibrator (2) and reflector (6) are arranged at opposing ends of the chamber (4).

4. The device of any preceding claim, wherein the pharmaceutical (6) is contained in the chamber (4), or a reservoir (10) in communication with the chamber (4).

5. The device of claim 4, wherein the reservoir (10) is located adjacent to an antinode of the standing wave pattern.

6. The device of claim 5, wherein at least one node and at least one antinode of the standing wave pattern are arranged such that a discrete amount of pharmaceutical (3) may be metered out of the reservoir (10) and come to rest at a junction between the node and antinode.

7. The device of any preceding claim, wherein the reflector has an acoustic impedance such that a portion of the acoustic energy produced by the vibrator is transmitted through the reflector, and wherein the transmission of a portion of the acoustic energy through the reflector preferably creates an acoustic energy interference pattern with shifting nodes and antinodes.

8. A method for nebulizing a dry powder pharmaceutical (3), said method comprising the steps of:
providing a drug chamber (4) comprising at least one inlet hole (7) and at least one outlet hole (8);
arranging a vibrator (2) and a reflector (6) at opposing ends of the drug chamber (4);
directing acoustic energy produced by the vibrator (2) to the reflector (6) such that at least a portion of the acoustic energy is reflected back to the vibrator (2);
introducing a dry powder pharmaceutical (3) into the drug chamber (4);
wherein the dry powder pharmaceutical (3) is aerosolized into a standing wave pattern created by the reflected acoustic energy, and a flow of air is created within the chamber (4) from the inlet hole (7) to the outlet hole (8) for carrying the dry powder pharmaceutical (3) from the drug chamber (4), wherein the at least one inlet hole (7) is adjacent to at least one node created by the standing wave pattern and/or the at least one outlet hole (8) is adjacent to at least one antinode created by the standing wave pattern.

9. The method of claim 8, wherein the drug chamber (4) is tube-shaped.

10. The method of claim 8 or claim 9, wherein the pharmaceutical (3) is introduced into the drug chamber (4) from a reservoir (10) in communication with the chamber (4), which reservoir (10) preferably is located adjacent to at least one antinode created by the standing wave pattern.

11. The method of claim 10, wherein a discrete amount of pharmaceutical (3) is metered out of the reservoir (10) into a junction between at least one node and at least one antinode created by the standing wave pattern.

12. The method of any of claims 8 to 11, wherein a portion of the acoustic energy is transmitted through the reflector (6).

## Patentansprüche

1. Inhalationsvorrichtung, umfassend eine Arzneimittelkammer (4) zum Beinhalten eines Trockenpulverpharmazeutikums (3);
eine Vibriervorrichtung (2) zum Aerosolisieren des Pharmazeutikums (3) in der Arzneimittelkammer (4); und
einen Wellenreflektor (6), wobei die Arzneimittelkammer (4) mindestens ein Einlassloch (7) und mindestens ein Auslassloch (8) umfasst, über die aerosolisiertes Pharmazeutikum ausgestoßen werden kann; und wobei die Vorrichtung so konfiguriert ist, dass durch akustische Energie ein stehendes Wellenmuster geschaffen wird, das durch die Vibriervorrichtung (2) erzeugt und durch den Reflektor (6) mindestens teilweise zurück zur Vibriervorrichtung (2) reflektiert wird, so dass Luft in das Einlassloch (7) und aus dem Auslassloch (8) gezogen werden kann, wobei durch die Vibriervorrichtung (2) erzeugte akustische Energie ausreicht, um einen Strom in der Arzneimittelkammer (4) zu schaffen, der in der Lage ist, das Pharmazeutikum (3) aus dem Auslassloch (8) zu tragen, und
**dadurch gekennzeichnet, dass**
sich das mindestens eine Einlassloch (7) angrenzend an einen Knoten des stehenden Wellenmusters befindet und/oder sich das mindestens eine Auslassloch (8) angrenzend an einen Bauch des stehenden Wellenmusters befindet.

2. Vorrichtung nach Anspruch 1, wobei die Vibriervorrichtung (2) einen piezoelektrischen Wandler umfasst.

3. Vorrichtung nach Anspruch 1 oder 2, wobei die Arzneimittelkammer (4) schlauchförmig ist und/oder wobei die Vibriervorrichtung (2) und der Reflektor (6) an gegenüberliegenden Seiten der Kammer (4) angeordnet sind.

4. Vorrichtung nach einem der vorangehenden Ansprüche, wobei das Pharmazeutikum (6) in der Kammer (4) oder einem mit der Kammer (4) in Verbindung stehenden Behälter (10) enthalten ist.

5. Vorrichtung nach Anspruch 4, wobei sich der Behälter (10) angrenzend an einen Bauch des stehenden Wellenmusters befindet.

6. Vorrichtung nach Anspruch 5, wobei der mindestens eine Knoten und der mindestens eine Bauch des stehenden Wellenmusters so angeordnet sind, dass eine diskrete Menge von Pharmazeutikum (3) aus dem Behälter (10) dosiert werden kann und auf einem Übergang zwischen dem Knoten und dem Bauch zu ruhen kommt.

7. Vorrichtung nach einem der vorangehenden Ansprüche , wobei der Reflektor eine akustische Impedanz aufweist, so dass ein Teil der von der Vibriervorrichtung erzeugten akustischen Energie durch den Reflektor übertragen wird, und wobei die Übertragung eines Teils der akustischen Energie durch den Reflektor vorzugsweise ein akustisches Energieinterferenzmuster mit sich verschiebenden Knoten und Bäuchen schafft.

8. Verfahren zum Zerstäuben eines Trockenpulverpharmazeutikums (3), wobei das Verfahren die folgenden Schritte umfasst:
ein Bereitstellen einer Arzneimittelkammer (4), umfassend mindestens ein Einlassloch (7) und mindestens ein Auslassloch (8);
ein Anordnen einer Vibriervorrichtung (2) und eines Reflektors (6) an gegenüberliegenden Enden der Arzneimittelkammer (4);
ein Richten akustischer Energie, die durch die Vibriervorrichtung (2) erzeugt wird, zum Reflektor (6), so dass mindestens ein Teil der akustischen Energie zurück zur Vibriervorrichtung (2) reflektiert wird;
ein Einführen eines Trockenpulverpharmazeutikums (3) in die Arzneimittelkammer (4); wobei das Trockenpulverpharmazeutikum (3) in eine durch die reflektierte akustische Energie geschaffene stehende Welle aerosolisiert wird, und in der Kammer (4) vom Einlassloch (7) zum Auslassloch (8) ein Luftstrom geschaffen wird, um das Trockenpulverpharmazeutikum (3) von der Arzneimittelkammer (4) zu tragen, wobei das mindestens eine Einlassloch (7) an mindestens einen durch das stehende Wellenmuster geschaffenen Knoten angrenzt und/oder das mindestens eine Auslassloch (8) an mindestens einen durch das stehende Wellenmuster geschaffenen Bauch angrenzt.

9. Verfahren nach Anspruch 8, wobei die Arzneimittelkammer (4) schlauchförmig ist.

10. Verfahren nach Anspruch 8 oder Anspruch 9, wobei das Pharmazeutikum (3) von einem Behälter (10), der mit der Kammer (4) in Verbindung steht, in die Arzneimittelkammer (4) eingeführt wird, welcher Behälter (10) sich vorzugsweise angrenzend an mindestens einen durch das stehende Wellenmuster geschaffenen Bauch befindet.

11. Verfahren nach Anspruch 10, wobei eine diskrete Menge an Pharmazeutikum (3) aus dem Behälter (10) in einen Übergang zwischen mindestens einem Knoten und mindestens einen Bauch, die durch das stehende Wellenmuster geschaffen werden, dosiert wird.

12. Verfahren nach einem der Ansprüche 8 bis 11, wobei ein Teil der akustischen Energie durch den Reflektor (6) übertragen wird.

## Revendications

1. Dispositif d'inhalation comprenant une chambre de médicament (4) destinée à contenir un produit pharmaceutique pulvérulent sec (3) ;
un vibrateur (2) destiné à aérosoliser ledit produit pharmaceutique (3) dans la chambre de médicament (4) ; et
un déflecteur d'onde (6), ladite chambre de médicament (4) comprenant au moins un trou d'entrée (7) et au moins un trou de sortie (8) par lequel le produit pharmaceutique aérosolisé peut être éjecté ; et ledit dispositif étant conçu de sorte qu'un motif d'onde stationnaire soit créé par l'énergie acoustique qui est produite par le vibrateur (2) et renvoyée au moins en partie vers le vibrateur (2) par le déflecteur (6) de façon à ce que de l'air puisse être aspiré dans le trou d'entrée (7) et hors du trou de sortie (8), ladite énergie acoustique produite par le vibrateur (2) étant suffisante pour créer un écoulement dans la chambre de médicament (4) capable de transporter le produit pharmaceutique (3) hors du trou de sortie (8), et
**caractérisé en ce que**
ledit au moins un trou d'entrée (7) est situé adjacent à un nœud du motif d'onde stationnaire, et/ou ledit au moins un trou de sortie (8) est situé adjacent à un ventre du motif d'onde stationnaire.

2. Dispositif selon la revendication 1, ledit vibrateur (2) comprenant un transducteur piézoélectrique.

3. Dispositif selon la revendication 1 ou 2, ladite chambre de médicament (4) étant en forme de tube, et/ou ledit vibrateur (2) et ledit déflecteur (6) étant disposés à des extrémités opposées de la chambre (4).

4. Dispositif selon l'une quelconque des revendications précédentes, ledit produit pharmaceutique (6) étant contenu dans la chambre (4) ou un réservoir (10) en communication avec la chambre (4).

5. Dispositif selon la revendication 4, ledit réservoir (10) étant situé adjacent à un ventre du motif d'onde stationnaire.

6. Dispositif selon la revendication 5, au moins un nœud et au moins un ventre du motif d'onde stationnaire étant agencés de sorte qu'une quantité discrète de produit pharmaceutique (3) puisse être dosée hors du réservoir (10) et mise au repos au niveau de la jonction entre le nœud et le ventre.

7. Dispositif selon l'une quelconque des revendications précédentes, ledit déflecteur présentant une impédance acoustique telle qu'une partie de l'énergie acoustique produite par le vibrateur est transmise par l'intermédiaire du déflecteur, et ladite transmission d'une partie de l'énergie acoustique par l'intermédiaire du déflecteur créant de préférence un motif d'interférence d'énergie acoustique avec des nœuds et des ventres se décalant.

8. Procédé de nébulisation d'un produit pharmaceutique pulvérulent sec (3), ledit procédé comprenant les étapes de :
fourniture d'une chambre de médicament (4) comprenant au moins un trou d'entrée (7) et au moins un trou de sortie (8) ;
l'agencement d'un vibrateur (2) et d'un déflecteur (6) à des extrémités opposées de la chambre de médicament (4) ;
l'orientation de l'énergie acoustique produite par le vibrateur (2) vers le déflecteur (6) de sorte qu'au moins une partie de l'énergie acoustique soit renvoyée vers le vibrateur (2) ;
l'introduction d'un produit pharmaceutique pulvérulent sec (3) dans la chambre de médicament (4) ;
ledit produit pharmaceutique pulvérulent sec (3) étant aérosolisé en un motif d'onde stationnaire créé par l'énergie acoustique réfléchie, et un écoulement d'air étant créé dans la chambre (4) du trou d'entrée (7) au trou de sortie (8) pour transporter le produit pharmaceutique pulvérulent sec (3) depuis la chambre de médicament (4), ledit au moins un trou d'entrée (7) étant adjacent à au moins un nœud créé par le motif d'onde stationnaire et/ou ledit au moins un trou de sortie (8) étant adjacent à au moins un ventre créé par le motif d'onde stationnaire.

9. Procédé selon la revendication 8, dans lequel la chambre de médicament (4) est en forme de tube.

10. Procédé selon la revendication 8 ou 9, dans lequel le produit pharmaceutique (3) est introduit dans la chambre de médicament (4) depuis un réservoir (10) en communication avec la chambre (4), lequel réservoir (10) est de préférence situé adjacent à au moins un ventre créé par le motif d'onde stationnaire.

11. Procédé selon la revendication 10, dans lequel une quantité discrète de produit pharmaceutique (3) est dosée hors du réservoir (10) dans une jonction entre au moins un nœud et au moins un ventre créés par le motif d'onde stationnaire.

12. Procédé selon l'une quelconque des revendications 8 à 11, dans lequel une partie de l'énergie acoustique est transmise par l'intermédiaire du déflecteur (6).
